# EUROPEAN PATENT SPECIFICATION

(11) **EP 2 735 260 B1**
(45) Date of publication and mention of the grant of the patent: **05.06.2019**
(21) Application number: 13818970.9
(22) Date of filing: 11.04.2013
(51) Int. Cl.: A61B 1/00, G02B 23/24

(54) **ENDOSCOPE**
ENDOSKOP
ENDOSCOPE

(30) Priority: 23.08.2012 JP 2012184348
(43) Date of publication of application: 28.05.2014
(73) Proprietor: Olympus Corporation, Tokyo 192-8507 (JP)
(72) Inventor: HAMAZAKI, Masanori, Hachioji-shi, Tokyo 192-8507 (JP); WATANABE, Takanori, Hachioji-shi, Tokyo 192-8507 (JP)
(74) Representative: Schicker, Silvia
(86) International application number: PCT/JP2013/060958
(87) International publication number: WO 2014/030385

(56) References cited:
- EP-A1- 1 880 656
- EP-A1- 2 494 909
- JP-A- 2003 210 388
- JP-A- 2006 187 546
- JP-A- 2011 120 863
- JP-A- 2011 255 088
- US-A1- 2011 112 363
- US-A1- 2011 306 838

## Description

### Technical Field

The present invention relates to a structure of a distal end face of an insertion portion in an endoscope.

### Background Art

Various technologies have been proposed for endoscopes which relate to the cleanability and water draining properties of an objective lens surface that serves as an observation window that is provided at a distal end portion of an endoscope.

For example, Japanese Patent Application Laid-Open Publication No. 2011-120863 discloses technology for an endoscope according to which an annular convex portion is provided around an observation window, an inclined face that inclines in an outer diameter direction and a vertical face are formed in the annular convex portion, and drops of water generated as the result of water feeding from an air/water feeding nozzle are received by the vertical face thereby to prevent the drops of water from flowing onto the observation window.

Further, for example, Japanese Patent Application Laid-Open Publication No. 2011-255088 discloses technology for an endoscope according to which an observation window base portion is provided around an observation window, and by forming the observation window base portion in a streamline shape with respect to a flow of cleaning fluid, the fluid is swiftly moved from a position over the observation window.

However, in the configuration of the endoscope disclosed in Japanese Patent Application Laid-Open Publication No. 2011-120863, the vertical face is formed in the annular convex portion, thereby forming a stepped portion. This causes a problem that such an endoscope is difficult to be applied especially to an endoscope for medical use which is configured to be introduced into a living body.
Furthermore, there is also a problem that the stepped portion causes a difficulty in cleaning with a brush before and after using the endoscope.

In addition, since the observation window base portion is provided in the configuration of the endoscope disclosed in the Japanese Patent Application Laid-Open Publication No. 2011-255088, fluid remains on the observation window base portion. Such remained fluid is likely to go back to the observation window, which problematically disturbs a field of view.

From document US 2011/0112363 A1, a head section for an inserter of an endoscope is known, in which a vertical plane stops backflow of a water drop towards an observation window, so as to prevent that the water drop reaches the top surface of the observation window.

Document US 2011/030683 A1 discloses an endoscope which is capable of letting a liquid drain off to a satisfactory level at the time of cleaning by forming an outer peripheral shape of an observation window base part into a streamline shape with respect to a flow of a cleaning fluid jetted from a nozzle.

The present invention has been achieved in view of the above-described problems, and an object of the present invention is to provide an endoscope which further improves cleanability and water draining property of an observation window composed of an objective lens.

### Disclosure of Invention

### Means for Solving the Problem

In order to achieve the above-described object, an endoscope according to claim 1 is suggested.
According to one example, an endoscope includes a window portion and an air/water feeding nozzle at a distal end of an insertion portion, the endoscope including: a flat portion that is provided with the window portion and the air/water feeding nozzle; the window portion that protrudes by a predetermined height from the flat portion; the air/water feeding nozzle that is provided facing the window portion from the flat portion, and that ejects a fluid toward a surface of the window portion; and an inclined portion that is formed at a circumferential edge portion of the window portion; wherein, in angles of inclination with respect to the flat portion of the inclined portion, in comparison to a first angle of elevation that is an angle in a direction along a first axis that passes through a center of the window portion in an ejection direction of the fluid that is ejected from the air/water feeding nozzle, a second angle of elevation that is an angle in a direction along a second axis that is orthogonal to the first axis at the center of the window portion is made a larger angle.

The endoscope of the present invention that is configured according to claim 1 can further improve the cleanability and water draining property of the window portion.

### Brief Description of the Drawings

Fig. 1 is an overall configuration diagram of an endoscope apparatus having an endoscope according to one aspect of the present invention;
Fig. 2 is a front view of a distal end portion as seen from a distal end face side according to the aspect of the present invention;
Fig. 3 is a cross-sectional view along a line III-III in Fig. 2 according to the aspect of the present invention;
Fig. 4 is a cross-sectional view along a line IV-O-IV in Fig. 2 according to the aspect of the present invention;
Fig. 5 is a plan view showing an air/water feeding nozzle and an observation window, which is a front view of the distal end portion as seen from the distal end face side according to the aspect of the present invention;
Fig. 6 is a cross-sectional view along a line VI-VI in Fig. 5 according to the aspect of the present invention;
Fig. 7 is a cross-sectional view along a line VII-VII in Fig. 5 according to the aspect of the present invention;
Fig. 8 is a plan view showing an air/water feeding nozzle, an observation window, and an inclined portion according to the aspect of the present invention;
Fig. 9 is a cross-sectional view of a distal end portion in which an inclined portion is a simple tapered structure according to the aspect of the present invention;
Fig. 10 is a cross-sectional view of a distal end portion in which an inclined portion has the present configuration according to the aspect of the present invention;
Fig. 11 is a graph showing a measurement result with respect to a flow rate of a fluid at a point A in Fig. 9 and Fig. 10 according to the aspect of the present invention;
Fig. 12 is a graph showing a measurement result with respect to a flow rate of a fluid at a point B in Fig. 9 and Fig. 10 according to the aspect of the present invention; and
Fig. 13 is a graph showing a measurement result with respect to a flow rate of a fluid at a point C in Fig. 9 and Fig. 10 according to the aspect of the present invention.

### Best Mode for Carrying Out the Invention

Hereunder, an endoscope that is the present invention is described. It should be noted that, in the following description, drawings that are based on each embodiment are schematic ones in which a relationship between a thickness and a width of each portion, thickness ratios of the respective portions and the like are different from those of actual portions, and the drawings may include portions in which dimensional relationships and ratios are different from one another.

An embodiment of the present invention is described hereunder with reference to the drawings.

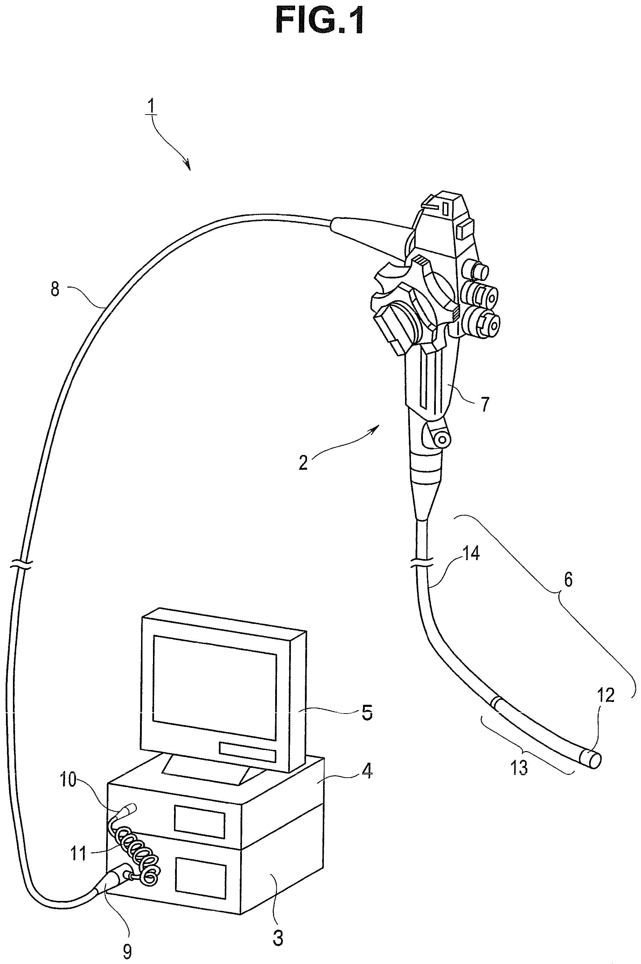

Fig. 1 is an overall configuration diagram of an endoscope apparatus having the endoscope of the present invention. As shown in Fig. 1, an endoscope apparatus 1 includes, as principal parts, an endoscope 2, a light source apparatus 3 that supplies an illuminating light, a video processor 4 that performs processing for an electrical signal that drives an image pickup apparatus to generate a video signal, and a monitor 5 that is a display apparatus that receives the video signal and displays an endoscopic image.

The endoscope 2 includes an insertion portion 6 that is inserted into a body cavity, an operation portion 7, and a universal cord 8. A connector 9 that is detachably connected to the light source apparatus 3 is provided at a distal end of the universal cord 8 whose proximal end portion is coupled to the operation portion 7. An electric cable 11 having, at a distal end portion, an electrical connector 10 that is connected to the video processor 4 extends from the connector 9. The insertion portion 6 includes a distal end portion 12, a bending portion 13, and a flexible portion 14 that are connected in series in that order from the distal end side.

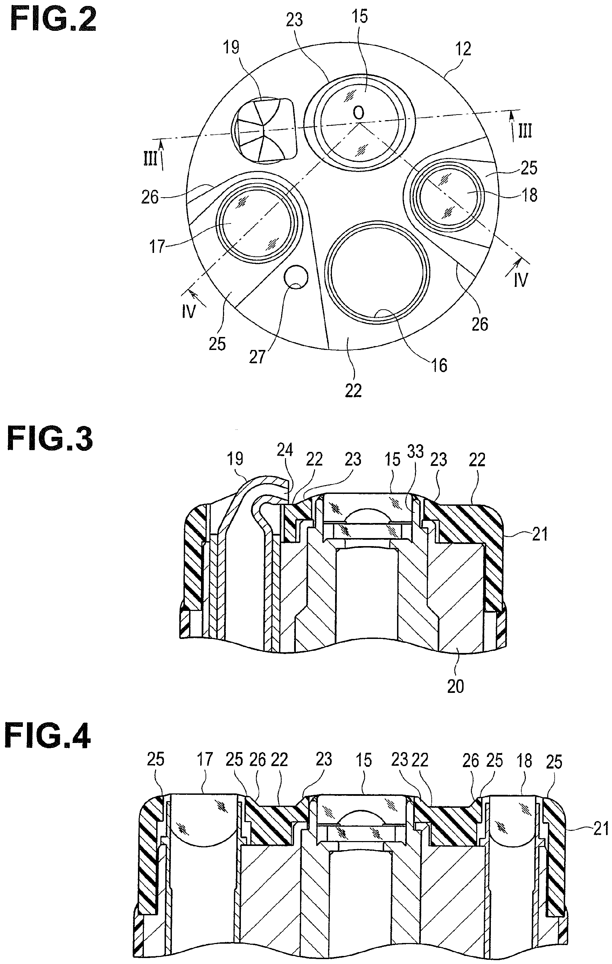

Fig. 2 is a front view of the distal end portion as seen from the distal end face side. As shown in Fig. 2, as viewed facing of a paper surface, an observation window 15 of a window portion including an objective lens is arranged toward an upper side, and a suction channel 16 is arranged toward a lower side on the distal end face of the distal end portion 12. In addition, a large illuminating window 17 including a large illumination lens is arranged toward a left side, and a small illuminating window 18 including a small illumination lens is arranged toward a right side. Further, a discharge port of an air/water feeding nozzle 19 is arranged facing toward the observation window 15 at a position that is adjacent to the upper side on the paper surface of the large illuminating window 17. The aforementioned small illuminating window 18 is provided adjacent to the observation window 15 in a diagonally lower right direction. Further, a forward water feeding channel 27 is arranged in a vicinity of the large illuminating window 17.

Fig. 3 is a cross-sectional view along a line III-III in Fig. 2 that shows a cross section from the air/water feeding nozzle 19 to the observation window 15. As shown in Fig. 3, a distal end rigid member 20 that fixes an objective lens support barrel 33 is provided in the distal end portion 12, and a distal end cover 21 is covered thereon. A flat portion 22 that forms a reference surface is formed on the distal end face of the distal end cover 21. The flat portion 22 occupies a major portion of the distal end face of the insertion portion 6. Note that a distal-end constituent member of the present embodiment is formed by the distal end rigid member 20 and the distal end cover 21.

A surface of the observation window 15 protrudes, for example, by 0.3 mm with respect to the flat portion 22. In the distal end cover 21 around the observation window 15, an inclined portion 23 is provided from the flat portion 22 to a circumferential edge portion of the observation window 15. In short, the inclined portion 23 that has a tapered shape toward the outer circumferential edge of the observation window 15 is formed in the distal end cover 21 around the observation window 15.

An opening portion 24 of the air/water feeding nozzle 19 is arranged so as to ride on the flat portion 22. The suction channel 16 is also provided in the flat portion 22 as shown in Fig. 2. An image pickup unit including an image pickup device and a lens group of an observation optical system (neither of which is illustrated in the drawings) are provided in the insertion portion 6 on an inner side of the observation window 15.

Note that, although in the present embodiment, a configuration is adopted in which the observation window 15 is configured as a window portion that includes an objective lens, more specifically, an objective lens whose rear face (face on a proximal end side of the distal end portion 12) is a concave face and whose front face is a flat face, the present invention is not limited thereto, and the objective lens may be any combination of a rear face that is a concave face, a convex face, or a flat face and a front face that is a convex face or a flat face, and the observation window 15 may also be a so-called "cover glass" in which both front and rear faces are flat faces.

Further, although in the present embodiment, the flat portion 22 and the inclined portion 23 are provided in the distal end face of the distal end cover 21, the present invention is not limited thereto, and for example, the flat portion 22 and the inclined portion 23 may be provided in the distal end rigid member 20 in an endoscope that does not have the distal end cover 21, and furthermore, the inclined portion 23 may be formed by the objective lens support barrel 33.

Fig. 4 is a cross-sectional view along a line IV-O-IV in Fig. 2 that shows a cross section from the large illuminating window 17 to the small illuminating window 18 that passes through a center O of the observation window 15. The large illuminating window 17 and the small illuminating window 18 protrude, for example, by 0.3 mm with respect to the flat portion 22 that forms the reference surface, and have a height that is equal to that of the observation window 15.

Planoconvex faces 25 that protrude, for example, by 0.3 mm with respect to the flat portion 22 are also formed in the distal end cover 21 around the large illuminating window 17 and the small illuminating window 18. In each planoconvex face 25, a boundary portion with the outer circumference of the distal end cover 21 is R-chamfered, and an inclined wall 26 is formed at a boundary with the flat portion 22.

A configuration of the inclined portion 23 of the present embodiment will now be described in detail.

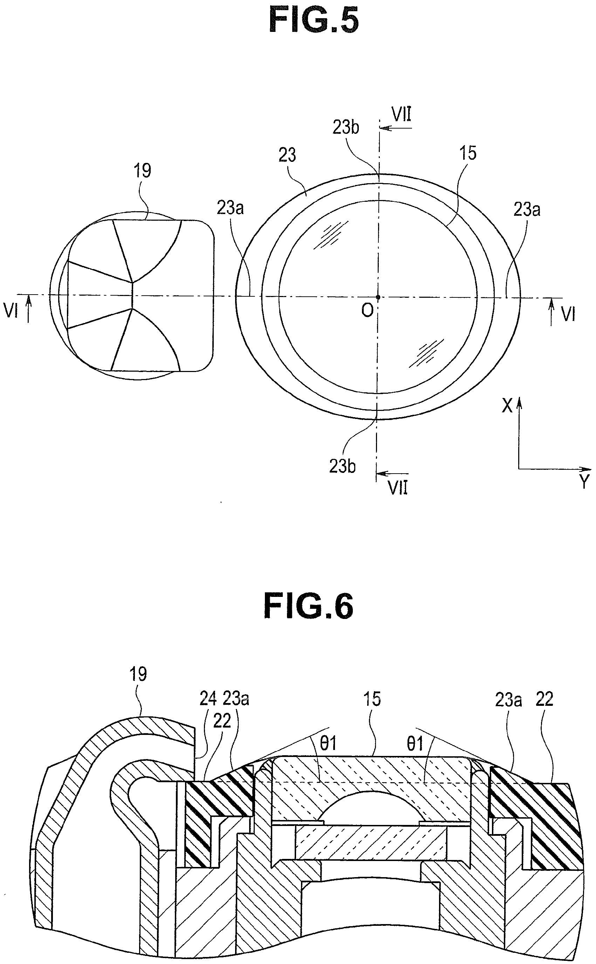

Fig. 5 is a plan view showing the air/water feeding nozzle and the observation window, which is a front view of the distal end portion as seen from the distal end face side. An external contour shape of the inclined portion 23 that is formed around the observation window 15 is an elliptical shape whose diameter is expanded in a lateral direction (direction of a longitudinal axis that passes through the center O of the observation window 15 along a Y-direction in the drawing, which may also be referred to as "Y-axis" in the following description) as viewed in the direction of the paper surface, and whose diameter is contracted in a vertical direction (short-axis direction that passes through the center O of the observation window 15 along an X-direction in the drawing, which may also be referred to as "X-axis" in the following description).

The air/water feeding nozzle 19 is disposed along the direction in which the diameter of the inclined portion 23 expands (Y-axis direction), and a fluid (a liquid flow for water feeding, air for air feeding or the like) from the air/water feeding nozzle 19 is ejected toward the observation window 15. Note that the air/water feeding nozzle 19 is arranged so that the Y-axis that passes through the center O of the observation window 15 passes through the center of the opening portion 24.

That is, the inclined portion 23 is configured to have a first inclined face 23a that is a face that slopes most gently from the flat portion 22 in the Y-axis direction that passes through the center O of the observation window 15 along the ejection direction of the fluid from the air/water feeding nozzle 19, and a second inclined face 23b that has a steeper slope than the first inclined face 23a in the X-axis direction that passes through the center O of the observation window 15 orthogonally to the ejection direction of the fluid from the air/water feeding nozzle 19. In the inclined portion 23, the second inclined face 23b has the steepest inclination from the flat portion 22.

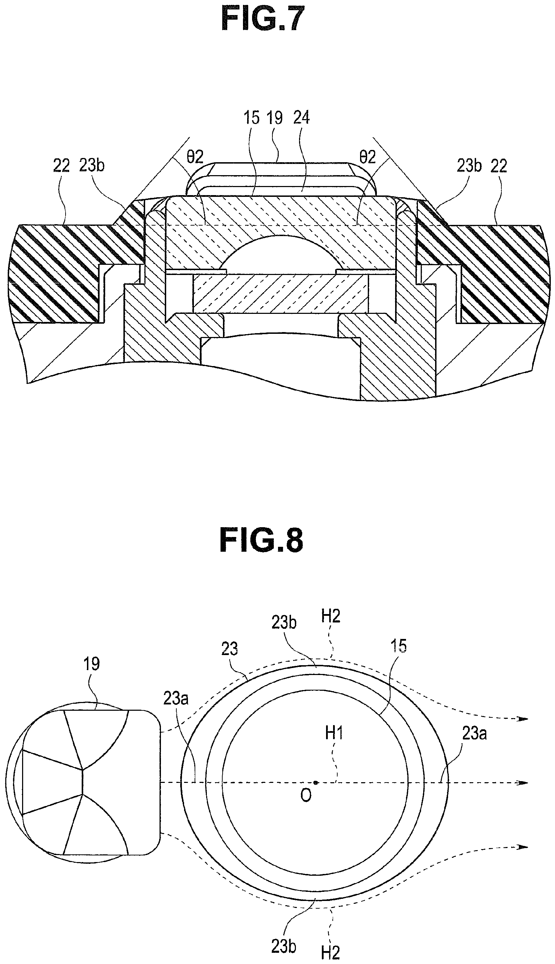

A structure of the inclined portion 23 will now be described in further detail. Fig. 6 is a cross-sectional view along a line VI-VI in Fig. 5. As shown in Fig. 6, the first inclined face 23a has a predetermined angle of elevation θ1 (angle formed by the first inclined face 23a and the flat portion 22) at which the first inclined face 23a inclines (slopes) from the flat portion 22. In contrast, as shown in Fig. 7 that is a cross-sectional view along a line VII-VII in Fig. 5, a predetermined angle of elevation θ2 (angle formed by the second inclined face 23b and the flat portion 22) at which the second inclined face 23b inclines (slopes) from the flat portion 22 is set to a larger angle than the predetermined angle of elevation θ1 of the first inclined face 23a (θ1 < θ2).

Here, the predetermined angle of elevation θ1 of the first inclined face 23a is set to, for example, 25° ± 10°, and the predetermined angle of elevation θ2 of the second inclined face 23b is set to, for example, 40° ± 10°. Note that a relationship between the predetermined angle of elevation θ1 of the first inclined face 23a and the predetermined angle of elevation θ2 of the second inclined face 23b is set so that the angle of elevation θ2 is always greater than the angle of elevation θ1 (θ1 < θ2). Further, a continuous slope (tapered face) is formed between the first inclined face 23a and the second inclined face 23b of the inclined portion 23 so that the slope between the respective predetermined angles of elevation θ1 and θ2 continuously changes.

According to the endoscope 2 of the present embodiment configured as described above, the configuration is such that fluid is ejected from the air/water feeding nozzle 19, and the fluid spreads out over the entire surface of the observation window 15 by means of the inclined portion 23. Fig. 8 is a plan view illustrating the air/water feeding nozzle, the observation window, and the inclined portion. As shown in Fig. 8, the flow rate of fluid H1 that passes over the first inclined face 23a and through the center O of the observation window 15 is the fastest among the fluid from the air/water feeding nozzle 19. On the other hand, because fluid H2 on each of the second inclined face 23b sides that are orthogonal to the ejection direction of the air/water feeding nozzle 19 is separated by a long distance from the center O of the observation window 15, among the fluid from the air/water feeding nozzle 19, the flow rate of the fluid H2 is inevitably slower than that of the fluid H1. In addition, for example, in a case where θ1 = θ2 in which the angle of elevation θ2 of the second inclined face 23b is identical to the first angle of elevation θ1, in comparison to the case where θ1 < θ2, the cross-sectional area of the second inclined face 23b would increase and would be liable to act as a barrier to the fluid H2, and thus the flow rate of the fluid H2 would be slower.

Therefore, as described above, according to the endoscope 2 of the present embodiment, in the inclined portion 23 that is formed at an edge portion around the observation window 15, the second inclined face 23b that is orthogonal to the ejection direction of the air/water feeding nozzle 19 is caused to incline (slope) more than the first inclined face 23a along the ejection direction of the air/water feeding nozzle 19, and a structure in which the cross-sectional area on the second inclined face 23b side is made smaller than the cross-sectional area on the first inclined face 23a, and not a simple tapered structure, and hence a decrease in the flow rate of the fluid H2 on the second inclined face 23b side is lessened (regulated).

That is, at the inclined portion 23 and the observation window 15, in the cross-sectional area that protrudes from the flat portion 22 along an axis that passes through the center O of the observation window 15, a cross-sectional area on the second inclined face 23b side that is orthogonal to the ejection direction of the air/water feeding nozzle 19 is smaller than a cross-sectional area on the first inclined face 23a side that is the ejection direction of the air/water feeding nozzle 19. Therefore, a reduction in the flow rate of the fluid H2 on the second inclined face 23b side can be lessened (regulated).

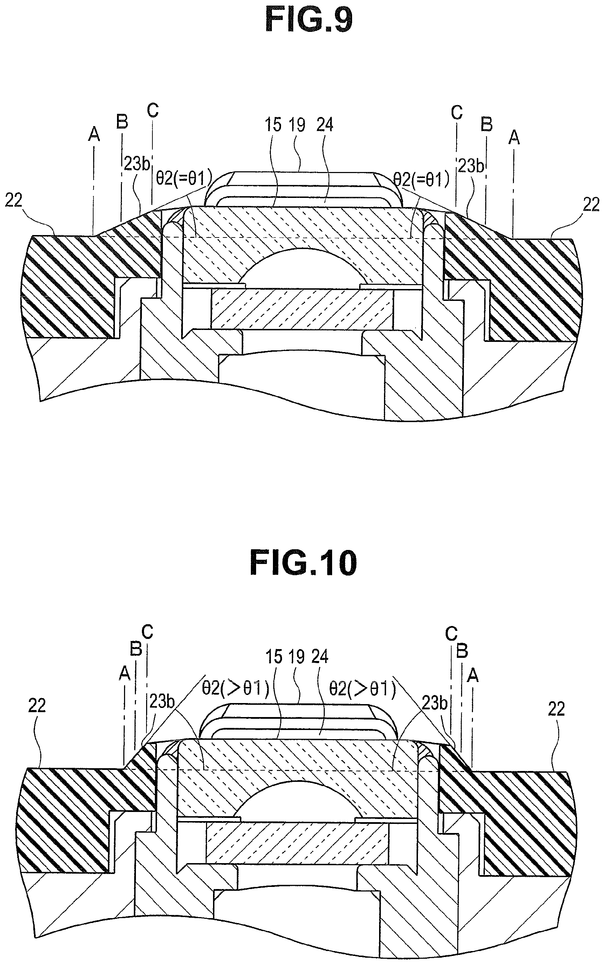

Fig. 9 is a cross-sectional view of a distal end portion in a case where an inclined portion is a simple tapered structure, and Fig. 10 is a cross-sectional view of the distal end portion of the inclined portion that has the present configuration. The flow rate of fluid from the air/water feeding nozzle 19 in the case where, as shown in Fig. 10, the predetermined angle of elevation θ2 of the second inclined face 23b is made larger than the predetermined angle of elevation θ1 of the first inclined face 23a (θ1 < θ2) that is the present configuration was measured with respect to the flow rate of fluid from the air/water feeding nozzle 19 in a case where a simple tapered structure was adopted in which, for example, as shown in Fig. 9, at the inclined portion 23, the predetermined angle of elevation θ2 of the second inclined face 23b was made the same angle as the predetermined angle of elevation θ1 of the first inclined face 23a (θ1 = θ2).

Note that, as shown in Fig. 9 and Fig. 10, the flow rate of fluid was measured at three places on the second inclined face 23b, namely, a point A at a boundary portion (vicinity of a base) with respect to the flat portion 22, a point B at approximately a center portion, and a point C at a boundary portion (vicinity of an apex) with respect to the observation window 15.

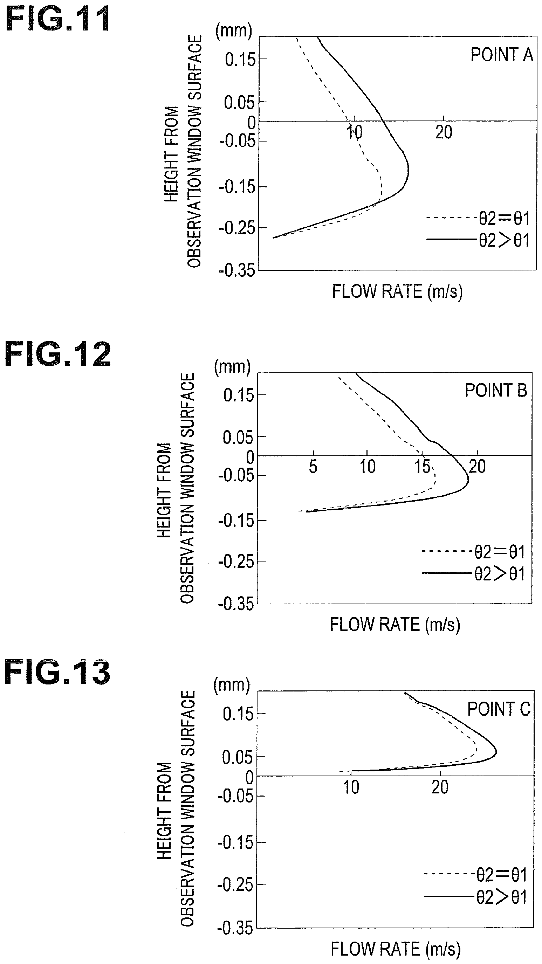

As shown in Fig. 11 to Fig. 13, results were obtained that showed that in comparison to the configuration in which the inclined portion 23 is formed to have a simple tapered structure as shown in Fig. 9, in the case of the inclined portion 23 according to the present configuration in which the predetermined angle of elevation θ2 of the second inclined face 23b is made larger than the predetermined angle of elevation θ1 of the first inclined face 23a (θ1 < θ2), the flow rate of fluid from the air/water feeding nozzle 19 is clearly faster at each of the three measurement points A, B and C. Note that Fig. 11 is a graph showing measurement results with respect to the flow rate of fluid at the point A in Fig. 9 and Fig. 10, Fig. 12 is a graph showing measurement results with respect to the flow rate of fluid at the point B in Fig. 9 and Fig. 10, and Fig. 13 is a graph showing measurement results with respect to the flow rate of fluid at the point C in Fig. 9 and Fig. 10.

More specifically, as shown in Fig. 11, with respect to the point A, a result was obtained to the effect that in the case of the configuration of the inclined portion 23 of the present configuration, the flow rate of fluid becomes faster than that of the simple tapered structure from a position at which a height from a lens face of the observation window 15 is around -0.20 mm, and thereafter the flow rate of the fluid is faster than that of the simple tapered structure even after passing a height of 0.15 mm from the lens face of the observation window 15.

Further, as shown in Fig. 12, with respect to the point B also, a result was obtained to the effect that in the case of the configuration of the inclined portion 23 of the present embodiment, the flow rate of fluid becomes faster than that of the simple tapered structure from a position at which the height from the lens face of the observation window 15 is around -0.12 mm, and thereafter the flow rate of the fluid is faster than that of the simple tapered structure even after passing the height of 0.15 mm from the lens face of the observation window 15.

Furthermore, as shown in Fig. 13, with respect to the point C also, a result was obtained to the effect that in the case of the configuration of the inclined portion 23 of the present embodiment, the flow rate of fluid becomes faster than that of the simple tapered structure from a position at which the height from the lens face of the observation window 15 is around 0.02 mm, and the flow rate of the fluid converges toward the same rate as that of the simple tapered structure from around a position at which the height of 0.15 mm from the lens face of the observation window 15 is passed.

Based on the foregoing, results are obtained to the effect that, in comparison to a simple tapered structure, the endoscope 2 of the present embodiment can regulate a decrease in the flow rate of fluid ejected from the air/water feeding nozzle 19 in the vicinity of the second inclined face 23b at which the angle of elevation θ2 is enlarged of the inclined portion 23 whose external contour shape is an elliptical shape. In other words, of the fluid ejected from the air/water feeding nozzle 19, a decrease in the flow rate of the fluid H2 on each of the second inclined face 23b sides that are orthogonal to the ejection direction of the air/water feeding nozzle 19 can be regulated to a significant degree relative to the flow rate of the fluid H1 that passes through the first inclined face 23a and passes through the center O of the observation window 15 that is illustrated in Fig. 8.

In this case, in the endoscope 2 of the present embodiment, an air/water feeding action is performed in the manner described hereunder.

First, in order to clean the observation window 15, in the endoscope 2, a water feeding operation is performed so as to eject a flow of liquid such as water from the opening portion 24 of the air/water feeding nozzle 19 onto the observation window 15. When this water feeding is performed, immediately after the liquid flow is ejected from the opening portion 24 of the air/water feeding nozzle 19, the liquid flow runs more easily onto the inclined portion 23 having the gentle (small) inclination (slope) adjacent to the opening portion 24 than the center of the observation window 15 including the first inclined face 23a, and spreads out extensively to extend over the entire surface of the observation window 15. Subsequently, the liquid flow that has run over the observation window 15 converges to a width that is the same level as the width of the opening portion 24 of the air/water feeding nozzle 19 while descending the inclined portion 23 that is separated from the opening portion 24 more than the center of the observation window 15 including the second inclined face 23b and the first inclined face 23a that is a water feeding destination of the inclined portion 23.

At this time, at the inclined portion 23 whose external contour shape is an elliptical shape of the endoscope 2, since the second inclined face 23b that is orthogonal to the feeding direction of the liquid flow ejected from the air/water feeding nozzle 19 is set to the predetermined angle of elevation θ2 which is a larger angle and has a steeper inclination (slope) than the predetermined angle of elevation θ1 of the first inclined face 23a whose inclination (slope) with respect to the flat portion 22 of the distal end portion 12 is gentle, a decrease in the flow rate of the liquid flow is lessened even at a place that is separated from the center O of the observation window 15 in a direction that is orthogonal to the feeding direction of the liquid flow. Consequently, dirt such as body fluid or mucous that attaches to the surface of the observation window 15 can be washed away easily by the liquid flow, and the cleanability of the entire surface of the observation window 15 improves.

Next, the endoscope 2 is switched from the water feeding operation to an air feeding operation, and air is fed from the opening portion 24 of the air/water feeding nozzle 19. When the air feeding is performed, water that remains on the surface of the observation window 15 is moved so as to be blown off the surface by the air.

That is, similarly to when performing water feeding, air ejected from the air/water feeding nozzle 19 flows more easily onto the inclined portion 23 having the gentle inclination (slope) adjacent to the opening portion 24 than the center of the observation window 15 including the first inclined face 23a, and temporarily spreads over the entire surface of the observation window 15, and after passing over the surface of the observation window 15, tends to converge when descending the inclined portion 23 that is separated from the opening portion 24 more than the center of the observation window 15 including the second inclined face 23b and the first inclined face 23a that is the air feeding destination of the inclined portion 23.

Consequently, when the operation is switched from water feeding to air feeding, drops of water on the surface of the observation window 15 spread out in a radial shape while moving so as to be blown off in mainly the air feeding direction, and are thus drained from the surface of the observation window 15.

At this time also, at the inclined portion 23 whose external contour shape is an elliptical shape of the endoscope 2, since the second inclined face 23b that is orthogonal to the feeding direction of the air ejected from the air/water feeding nozzle 19 is set to the predetermined angle of elevation θ2 which is a larger angle and has a steeper inclination (slope) than the predetermined angle of elevation θ1 of the first inclined face 23a whose inclination (slope) with respect to the flat portion 22 of the distal end portion 12 is gentle, a decrease in the flow rate of the air is lessened even at a place that is separated from the center O of the observation window 15 in a direction that is orthogonal to the feeding direction of the air. Consequently, remaining water is easily blown off from the entire observation window 15 by the air feeding, and the water draining property of the observation window 15 improves.

Thus, according to the endoscope 2 of the present embodiment, the observation window 15 is caused to protrude from the surface of the distal end portion 12, the inclined portion 23 whose external contour shape is an elliptical shape is formed around the observation window 15, it is easy for liquid and air that are fed in order to clean the observation window 15 to spread over the entire surface of the observation window 15, the manner in which liquid and air reach the surface of the observation window 15 is favorable, unwashed remnants of body fluid, mucous and the like that were attached to the distal end face of the observation window 15 are reduced thereby to improve the cleanability, and a water draining property with respect to water that remains after cleaning the observation window 15 by means of a liquid flow also improves.

Further, as shown in Fig. 2 and Fig. 4, the planoconvex faces 25 that protrude with respect to the flat portion 22 are formed around the large illuminating window 17 and the small illuminating window 18 also. If the planoconvex faces 25 around the large illuminating window 17 and the small illuminating window 18 and the inclined portion 23 formed around the observation window 15 were adjacent, a phenomenon would occur whereby a turbulent flow is generated in the fluid ejected from the air/water feeding nozzle 19 or whereby drops of water on the planoconvex faces 25 are drawn onto the surface of the observation window 15.

However, according to the endoscope 2 of the present embodiment, by adopting a configuration in which the shape of the inclined portion 23 around the observation window 15 is such that the external contour shape is an elliptical shape in which the outer diameter orthogonal to the ejection direction of fluid from the air/water feeding nozzle 19 is reduced, the distance by which the inclined portion 23, particularly the second inclined face 23b, is separated from the planoconvex faces 25 around the large illuminating window 17 and the small illuminating window 18 increases. In other words, the length of the flat portion 22 between the inclined portion 23, particularly the second inclined face 23b, and each planoconvex face 25 increases.

According to this configuration, since the inclined portion 23 formed around the observation window 15 and the planoconvex faces 25 around the large illuminating window 17 and the small illuminating window 18 do not come close to each other and a predetermined separation distance can be maintained therebetween, the occurrence of a phenomenon whereby a turbulent flow is generated in the fluid ejected from the air/water feeding nozzle 19 or whereby drops of water on the planoconvex faces 25 are drawn onto the surface of the observation window 15 can be reduced.

Note that the invention described in the foregoing embodiment is not limited to the embodiment and modification described above, and various modifications can be implemented within a range that does not deviate from the spirit and scope of the present invention in the implementing stage. Further, the above described embodiment includes inventions of various stages, and various inventions can be extracted by appropriately combining a plurality of the disclosed configuration requirements.

For example, if a problem to be solved by the invention can be solved and the described effects of the invention are obtained even after omitting some of the configuration requirements from the entire configuration requirements shown in the embodiment, then the configuration obtained by omitting the configuration requirements can be extracted as an invention.

## Claims

1. An endoscope (1), comprising:
a flat portion (22) that is provided at a distal end of an endoscope insertion portion (6);
a window portion that protrudes by a predetermined height along an insertion direction of the endoscope insertion portion (6) from the flat portion (22);
an air/water feeding nozzle (19) that is provided facing the window portion from the flat portion (22), and that ejects a fluid toward a surface of the window portion; and
an inclined portion (23) that is formed at a circumferential edge portion of the window portion;
wherein the inclined portion (23) includes a first angle of inclination (θ1) and a second angle of inclination (θ2) which are angles of inclination of the inclined portion (23) with respect to the flat portion (22), the first angle of inclination (θ1) being an angle in a direction along a first axis that passes through a center of the window portion and extends in an ejection direction of the fluid that is ejected from the air/water feeding nozzle (19), the second angle of inclination (θ2) being an angle in a direction along a second axis that is orthogonal to the first axis at the center of the window portion and set to a larger angle than the first angle of inclination, **characterized in that** the first and second angles of inclination (θ1, θ2) each defining oppositely arranged inclined faces (23a, 23b) along the respective first and second axis.

2. The endoscope (1) according to claim 1, wherein, with respect to the inclined portion (23), a cross-sectional area in a direction along the second axis is made smaller than a cross-sectional area in a direction along the first axis.

3. The endoscope (1) according to claim 1, wherein, with respect to a cross-sectional area comprising the inclined portion (23) and the window portion, a cross-sectional area of a portion protruding from the flat portion (22) in a direction along the second axis is made smaller than a cross-sectional area in a direction along the first axis.

4. The endoscope (1) according to claim 1, wherein the window portion is an observation window (15) for observation that has an objective lens.

5. The endoscope (1) according to claim 1, wherein:
the window portion is formed in a circular surface shape; and
an external contour shape of the inclined portion (23) is an elliptical shape.

6. The endoscope (1) according to claim 5, wherein the air/water feeding nozzle (19) is disposed so as to eject the fluid in a longitudinal axis direction of the elliptical inclined portion.

7. The endoscope (1) according to claim 1, wherein a continuous inclined face is formed between the first angle of inclination (θ1) and the second angle of inclination (θ2) of the inclined portion (23).

## Patentansprüche

1. Endoskop (1), das umfasst:
einen flachen Abschnitt (22), der an einem distalen Ende eines Endoskopeinführabschnitts (6) bereitgestellt ist;
einen Fensterabschnitt, der um eine vordefinierte Höhe entlang einer Einführrichtung des Endoskopeinführabschnitts (6) vom flachen Abschnitt (22) vorsteht;
ein Luft-/Wasserzufuhrdüse (19), die dem Fensterabschnitt zugewandt vom flachen Abschnitt (22) bereitgestellt ist und die ein Fluid hin zu einer Oberfläche des Fensterabschnitts ausstößt; und
einen geneigten Abschnitt (23), der an einem Umfangsrandabschnitt des Fensterabschnitts gebildet ist;
wobei der geneigte Abschnitt (23) einen ersten Neigungswinkel (θ1) und einen zweiten Neigungswinkel (θ2) umfasst, die Neigungswinkel des geneigten Abschnitts (23) in Bezug auf den flachen Abschnitt (22) sind, wobei der erste Neigungswinkel (θ1) ein Winkel in einer Richtung entlang einer ersten Achse ist, die durch eine Mitte des Fensterabschnitts verläuft und sich in eine Ausstoßrichtung des Fluids erstreckt, das aus der Luft-/Wasserzufuhrdüse (19) ausgestoßen wird, wobei der zweite Neigungswinkel (θ2) ein Winkel in einer Richtung entlang einer zweiten Achse ist, die orthogonal zur ersten Achse in der Mitte des Fensterabschnitts verläuft, und auf einen größeren Winkel als der erste Neigungswinkel eingestellt ist, **dadurch gekennzeichnet, dass** der erste und der zweite Neigungswinkel (θ1, θ2) jeweils gegenüberliegend angeordnete geneigte Flächen (23a, 23b) entlang der jeweiligen ersten und zweiten Achse definieren.

2. Endoskop (1) nach Anspruch 1, wobei eine Querschnittsfläche in einer Richtung entlang der zweiten Achse in Bezug auf den geneigten Abschnitt (23) kleiner als eine Querschnittsfläche in einer Richtung entlang der ersten Achse ausgestaltet ist.

3. Endoskop (1) nach Anspruch 1, wobei eine Querschnittsfläche eines Abschnitts, der vom flachen Abschnitt (22) vorsteht, in Bezug auf eine Querschnittsfläche, die den geneigten Abschnitt (23) und den Fensterabschnitt umfasst, in einer Richtung entlang der zweiten Achse kleiner als eine Querschnittsfläche in einer Richtung entlang der ersten Achse ausgestaltet ist.

4. Endoskop (1) nach Anspruch 1, wobei der Fensterabschnitt ein Beobachtungsfenster (15) zum Beobachten ist, das eine Objektivlinse aufweist.

5. Endoskop (1) nach Anspruch 1, wobei:
der Fensterabschnitt in einer kreisförmigen Oberflächenform ausgebildet ist; und
eine Außenkonturform des geneigten Abschnitts (23) eine elliptische Form ist.

6. Endoskop (1) nach Anspruch 5, wobei die Luft-/Wasserzufuhrdüse (19) so ausgelegt ist, dass sie das Fluid in einer Längsachsenrichtung des elliptischen geneigten Abschnitts ausstößt.

7. Endoskop (1) nach Anspruch 1, wobei eine durchgehende geneigte Fläche zwischen dem ersten Neigungswinkel (θ1) und dem zweiten Neigungswinkel (θ2) des geneigten Abschnitts (23) ausgebildet ist.

## Revendications

1. Endoscope (1), comprenant :
une partie plate (22) qui est prévue à une extrémité distale d'une partie d'insertion d'endoscope (6) ;
une partie fenêtre qui fait saillie d'une hauteur prédéterminée le long d'une direction d'insertion de la partie d'insertion d'endoscope (6) à partir de la partie plate (22) ;
une buse d'alimentation en air/eau (19) qui est prévue dirigée vers la partie fenêtre à partir de la partie plate (22), et qui éjecte un fluide vers une surface de la partie fenêtre ; et
une partie inclinée (23) qui est formée à une partie de bord circonférentiel de la partie fenêtre ;
la partie inclinée (23) comprenant un premier angle d'inclinaison (θ1) et un second angle d'inclinaison (θ2) qui sont des angles d'inclinaison de la partie inclinée (23) par rapport à la partie plate (22), le premier angle d'inclinaison (θ1) étant un angle dans une direction le long d'un premier axe qui passe par un centre de la partie fenêtre et qui s'étend dans une direction d'éjection du fluide qui est éjecté à partir de la buse d'alimentation en air/eau (19), le second angle d'inclinaison (θ2) étant un angle dans une direction le long d'un second axe qui est orthogonal au premier axe au niveau du centre de la partie fenêtre et défini comme pour être un angle plus grand que le premier angle d'inclinaison, **caractérisé par le fait que** les premier et second angles d'inclinaison (θ1, θ2) définissent chacun des faces inclinées agencées de manière opposée (23a, 23b) le long des premier et second axes respectifs.

2. Endoscope (1) selon la revendication 1, dans lequel, par rapport à la partie inclinée (23), une section transversale dans une direction le long du second axe est plus petite qu'une section transversale dans une direction le long du premier axe.

3. Endoscope (1) selon la revendication 1, dans lequel, par rapport à une section transversale comprenant la partie inclinée (23) et la partie fenêtre, une section transversale d'une partie faisant saillie à partir de la partie plate (22) dans une direction le long du second axe est plus petite qu'une section transversale dans une direction le long du premier axe.

4. Endoscope (1) selon la revendication 1, dans lequel la partie fenêtre est une fenêtre d'observation (15) pour une observation, qui a un objectif.

5. Endoscope (1) selon la revendication 1, dans lequel :
la partie fenêtre a une forme de surface circulaire ; et
une forme de contour externe de la partie inclinée (23) est une forme elliptique.

6. Endoscope (1) selon la revendication 5, dans lequel la buse d'alimentation en air/eau (19) est disposée de façon à éjecter le fluide dans une direction d'axe longitudinal de la partie inclinée elliptique.

7. Endoscope (1) selon la revendication 1, dans lequel une face inclinée continue est formée entre le premier angle d'inclinaison (θ1) et le second angle d'inclinaison (θ2) de la partie inclinée (23).
